# EUROPEAN PATENT SPECIFICATION

(11) **EP 4 225 297 B1**
(45) Date of publication and mention of the grant of the patent: **23.10.2024**
(21) Application number: 21794474.3
(22) Date of filing: 07.10.2021
(51) Int. Cl.: A61K 31/282, A61K 31/496, A61K 31/555, A61K 33/243, A61K 45/06, A61P 35/00

(54) **COMBINATION THERAPY FOR TREATING CANCER**
KOMBINATIONSTHERAPIE ZUR BEHANDLUNG VON KREBS
POLYTHÉRAPIE POUR LE TRAITEMENT DU CANCER

(30) Priority: 08.10.2020 US 202063089195 P
(43) Date of publication of application: 16.08.2023
(73) Proprietor: Astrazeneca AB, 151 85 Södertälje (SE)
(72) Inventor: LEO, Elisabetta, Cambridge Cambridgeshire CB2 0AA (GB)
(74) Representative: AstraZeneca Intellectual Property
(86) International application number: PCT/EP2021/077703
(87) International publication number: WO 2022/074124

(56) References cited:
- WO-A1-2021/013735
- ANONYMOUS: "History of Changes for Study: NCT04644068 - Study of AZD5305 as Monotherapy and in Combination With Anti-cancer Agents in Patients With Advanced Solid Malignancies (PETRA)", 19 November 2020 (2020-11-19), XP055875100, Retrieved from the Internet <URL:https://clinicaltrials.gov/ct2/history/NCT04644068?A=1&B=1&C=merged#StudyPageTop> [retrieved on 20211221]
- HU XIAO-LONG ET AL: "Identification of amentoflavone as a potent highly selective PARP-1 inhibitor and its potentiation on carboplatin in human non-small cell lung cancer", PHYTOMEDICINE, ELSEVIER, AMSTERDAM, NL, vol. 50, 5 September 2018 (2018-09-05), pages 88 - 98, XP085539127, ISSN: 0944-7113, DOI: 10.1016/J.PHYMED.2018.09.012
- STANISZEWSKA A D ET AL: "The novel PARP1-selective inhibitor, AZD5305, is efficacious as monotherapy and in combination with standard of care chemotherapy in the in vivo preclinical models", CANCER RESEARCH 20210701 AMERICAN ASSOCIATION FOR CANCER RESEARCH INC. NLD, vol. 81, no. 13 SUPPL, 1 July 2021 (2021-07-01), XP009532404, ISSN: 1538-7445
- ANONYMOUS: "Study Record Versions History of Changes for Study: NCT04644068 Study of AZD5305 as Monotherapy and in Combination With Anti-cancer Agents in Patients With Advanced Solid Malignancies (PETRA)", 19 November 2020 (2020-11-19), XP055875178, Retrieved from the Internet <URL:https://clinicaltrials.gov/ct2/history/NCT04644068?A=1&B=1&C=merged#StudyPageTop> [retrieved on 20211221]

## Description

### Background

While much progress has been made in the treatment of cancer, patients continue to need new and effective therapies.

### Summary

AZD5305 (5-[4-[(7-ethyl-6-oxo-5H-1,5-naphthyridin-3-yl)methyl]piperazin-1-yl]-N-methylpyridine-2-carboxamide) is a small molecule drug that acts by selectively inhibiting and trapping PARP1 at the sites of DNA single cell breaks (SSB). This both prevents the DNA repair and, during DNA replication, leads to the generation of the more deleterious DNA double strand breaks (DSB), when the DNA replication machinery collides with the PARP1-DNA non-covalent complexes. In contexts where the accurate DNA repair pathways are effective, such as in cells with a proficient homologous recombination repair (HRR), DSBs are accurately repaired. In contrast, in cells with deficiencies in the repair pathways, such as those with deleterious mutation in the BRCA genes, AZD5305 treatments lead to selective accumulation of genome instability which ultimately selectively kill cancer cells, while sparing normal cells.

Platinum chemotherapeutic agents are drugs used to treat cancer, frequently in the first line treatment setting. Examples of platinum chemotherapeutic agents include cisplatin, oxaliplatin, and carboplatin.

Hu Xiao-Long et al. (Phytomedicine, 50, 2018, p88-98) teaches the identification of amentoflavone as a potent highly selective PARP-1 inhibitor and its potentiation on carboplatin in human non-small cell lung cancer.

The present invention is directed to AZD5305, or a pharmaceutically acceptable salt thereof, for use in the treatment of cancer in a subject, wherein said treatment comprises the separate, sequential or simultaneous administration of i) said AZD5305, or a pharmaceutically acceptable salt thereof, and ii) a platinum chemotherapeutic agent, or a pharmaceutically acceptable salt thereof, to said subject.

The invention is further directed to a pharmaceutical product comprising i) AZD5305 or a pharmaceutically acceptable salt thereof, and ii) a platinum chemotherapeutic agent or a pharmaceutically acceptable salt thereof.

In some embodiments, the cancer is ovary, breast, pancreas, prostate, hematological, gastrointestinal such as gastric and colorectal, or lung cancer.

In some embodiments the cancer is homologous recombination deficient (HRD) cancer. For example, whether the cancer is HRD positive can be determined by Myriad Genetics myChoice^{®} HRD or myChoice^{®} HRD Plus assay.

In certain embodiments, the cancer cells comprise HRD gene mutation selected from *BRCA1, BRCA2, ATM, BRIP1, BARD1, CDK12, CHEK1, CHEK2, FANCL, PALB2, PPP2R2A, RAD51B, RAD51C, RAD51D,* and *RAD54L* gene mutation. In certain embodiments, the cancer cells comprise a *BRCA1,* a *BRCA2,* and/or an *ATM* gene mutation. In certain embodiments, the cancer cells comprise a *BRCA1* and/or a *BRCA2* gene mutation. For example, in certain embodiments, the cancer cells comprise a *tBRCA* gene mutation.

In certain embodiments, the cancer comprises homologous recombination deficiency (HRD)-positive status defined by a deleterious or suspected deleterious BRCA mutation and/or genomic instability.

In certain embodiments, the cancer is ovarian cancer or breast cancer. In certain embodiments, the cancer is ovarian cancer. In certain embodiments, the cancer is advanced epithelial ovarian cancer. In certain embodiments, the cancer is high-grade serous ovarian cancer. In certain embodiments, the cancer is high-grade endometrioid ovarian cancer. In certain embodiments, the cancer is epithelial ovarian cancer comprising a *gBRCA1* or a *gBRCA2* mutation. In certain embodiments, the cancer is fallopian tube cancer. In certain embodiments, the cancer is primary peritoneal cancer.

In certain embodiments, the cancer is ovarian (such as advanced epithelial ovarian), fallopian tube, or primary peritoneal cancer.

In certain embodiments, the cancer is ovarian (such as advanced epithelial ovarian), fallopian tube, or primary peritoneal cancer, the cancer comprising homologous recombination deficiency (HRD)-positive status defined by a deleterious or suspected deleterious BRCA mutation and/or genomic instability.

In certain embodiments, the cancer is breast cancer. In certain embodiments the cancer is triple negative breast cancer.

In some embodiments, the cancer is platinum-resistant.

In some embodiments, disclosed is a kit comprising a first pharmaceutical composition comprising AZD5305 or a pharmaceutically acceptable salt thereof and a pharmaceutically acceptable carrier; and a second pharmaceutical composition comprising a platinum chemotherapeutic agent or a pharmaceutically acceptable salt thereof and instructions for use.

### Brief Descriptions of the Drawing

**Figure 1** illustrates the body weight change of mice in a PDX model (HBCx-9) treated with AZD5305 monotherapy, carboplatin monotherapy, and in combination.
**Figure 2** illustrates the anti-tumour activity of AZD5305 in combination with carboplatin in a PDX model (HBCx-9).
**Figure 3** illustrates the anti-tumour activity of AZD5305 in combination with carboplatin in a PDX model (HBCx-9). Individual animal data is shown.
**Figure 4** shows an X-ray powder diffraction of AZD5305 Form A
**Figure 5** illustrates the body weight change of mice in a xenograft model (SUM149PT) treated with AZD5305 monotherapy, carboplatin monotherapy, and in combination.
**Figure 6** illustrates the anti-tumour activity of AZD5305 in combination with carboplatin in a xenograft model (SUM149PT).
**Figure 7** illustrates the body weight change of mice in a PDX model (HBCx-9) treated with AZD5305 monotherapy, carboplatin monotherapy, and in combination.
**Figure 8** illustrates the anti-tumour activity of AZD5305 in combination with carboplatin in a PDX model (HBCx-9).
**Figure 9** illustrates the anti-tumour activity of AZD5305 in combination with carboplatin following cessation of treatment in a PDX model (HBCx-9) treated with AZD5305 monotherapy, carboplatin monotherapy, and in combination.

### Detailed Description

The references to methods of treatment in the detailed description of the invention in this description are to be interpreted as references to the compounds, pharmaceutical compositions and medicaments of the present invention for use in a method for treatment of the human (or animal) body by therapy.

In some embodiments, disclosed is a method of treating cancer by a combination therapy of AZD5305 and a platinum chemotherapeutic agent. In some embodiments, the method comprises administering to a subject in need thereof a first amount of AZD5305 or a pharmaceutically acceptable salt thereof and a second amount of a platinum chemotherapeutic agent or a pharmaceutically acceptable salt thereof, wherein the first amount and the second amount together comprises a therapeutically effective amount. In some embodiments, the platinum chemotherapeutic agent includes any one of carboplatin, cisplatin and oxaliplatin. In some embodiments, the platinum chemotherapeutic agent includes carboplatin. In an embodiment, the platinum chemotherapeutic agent or a pharmaceutically acceptable salt thereof is administered first, and AZD5305 or a pharmaceutically acceptable salt thereof is administered second. In an embodiment, AZD5305 or a pharmaceutically acceptable salt is administered first, and the platinum chemotherapeutic agent or a pharmaceutically acceptable salt is administered second.

The term "AZD5305" refers to a compound with the chemical name of 5-[4-[(7-ethyl-6-oxo-5H-1,5-naphthyridin-3-yl)methyl]piperazin-1-yl]-N-methyl-pyridine-2-carboxamide and structure shown below:

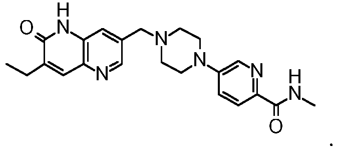

Preparation of AZD5305 is disclosed herein (see Example 1). In some embodiments, a free base of AZD5305, is administered to a subject. In some embodiments, a pharmaceutically acceptable salt of AZD5305 is administered to a subject. In some embodiments, a crystalline AZD5305 is administered to a subject. In some embodiments, crystalline Form A AZD5305 is administered to a subject.

The term "platinum-containing chemotherapeutic agent" includes drugs that contain the metal platinum, such as cisplatin, carboplatin, and oxaliplatin.

In some embodiments, AZD5305 or a pharmaceutically acceptable salt thereof and a platinum chemotherapeutic agent or a pharmaceutically acceptable salt thereof are administered separately, sequentially or simultaneously. In an embodiment, the platinum chemotherapeutic agent or a pharmaceutically acceptable salt thereof is administered first, and AZD5305 or a pharmaceutically acceptable salt thereof is administered second. In an embodiment, AZD5305 or a pharmaceutically acceptable salt is administered first, and the platinum chemotherapeutic agent or a pharmaceutically acceptable salt is administered second.

In some embodiments, AZD5305 or a pharmaceutically acceptable salt thereof and a platinum chemotherapeutic agent or a pharmaceutically acceptable salt thereof are administered separately, sequentially or simultaneously in a treatment cycle.

A "cycle", "treatment cycle" or "dosing schedule", as used herein, refers to a period of combination treatment that is repeated on a regular schedule. For example, the treatment can be given for one week, two weeks, or three weeks wherein AZD5305 and a platinum chemotherapeutic agent are administered in a coordinated fashion. In some embodiments, a treatment cycle is about 1 week to about 3 months. In some embodiments, a treatment cycle is about 5 days to about 1 month. In some embodiments, a treatment cycle is about 1 week to about 3 weeks. In some embodiments, a treatment cycle is about 1 week, about 10 days, about 2 weeks, about 3 weeks, about 4 weeks, about 2 months, or about 3 months.

In some embodiments, AZD5305 or a pharmaceutically acceptable salt thereof and a platinum chemotherapeutic agent or a pharmaceutically acceptable salt thereof are administered to the human subject in one or more treatment cycles, e.g., a treatment course. A "treatment course" comprises multiple treatment cycles, which can be repeated on a regular schedule, or adjusted as a tapered schedule as the patient's disease progression is monitored. For example, a patient's treatment cycles can have longer periods of treatment and/or shorter periods of rest at the beginning of a treatment course (e.g., when the patient is first diagnosed), and as the cancer enters remission, the rest period lengthens, thereby increasing the length of one treatment cycle. The period of time for treatment and rest in a treatment cycle, the number of treatment cycles, and the length of time for the treatment course can be determined and adjusted throughout the treatment course by the skilled artisan based on the patient's disease progression, treatment tolerance, and prognosis. In some embodiments, the method comprises 1 to 10 treatment cycles. In some embodiments, the method comprises 2 to 8 treatment cycles.

In some embodiments, AZD5305 or a pharmaceutically acceptable salt thereof is administered orally. In some embodiments, AZD5305 or a pharmaceutically acceptable salt thereof is in capsule dosage form. In some embodiments, AZD5305 or a pharmaceutically acceptable salt thereof is in tablet dosage form.

The language "treat," "treating" and "treatment" includes the reduction or inhibition of enzyme or protein activity related to, PARP or cancer in a subject, amelioration of one or more symptoms of cancer in a subject, or the slowing or delaying of progression of cancer in a subject. The language "treat," "treating" and "treatment" also includes the reduction or inhibition of the growth of a tumor or proliferation of cancerous cells in a subject.

The language "inhibit," "inhibition" or "inhibiting" includes a decrease in the baseline activity of a biological activity or process.

The term "cancer" includes, but is not limited to a disease caused by an uncontrolled division of abnormal cells in a part of the body. In some embodiments, the cancer includes cancers that are susceptible to treatment with PARP inhibitors (*e.g*., AZD5305). In some embodiments, the cancer is ovarian cancer, breast cancer, pancreatic cancer, and prostate cancer. In some embodiments the cancer is hematological, gastrointestinal such as gastric and colorectal, or lung cancer. In some embodiments, the cancer is relapsed or refractory cancer. In some embodiments, the cancer is platinum-resistant cancer.

The language "pharmaceutical composition" includes compositions comprising an active ingredient and a pharmaceutically acceptable excipient, carrier or diluent, wherein the active ingredient is AZD5305 or a pharmaceutically acceptable salt thereof, or a platinum chemotherapeutic agent or a pharmaceutically acceptable salt thereof.

In this specification, unless otherwise stated, the term "pharmaceutically acceptable" as used herein refers to those compounds, materials, compositions, salts, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

The language "pharmaceutically acceptable excipient, carrier or diluent" includes compounds, materials, compositions, and/or dosage forms which are, within the scope of sound medical judgment, suitable for use in contact with the tissues of human beings and animals without excessive toxicity, irritation, allergic response, or other problem or complication, as ascertained by one of skill in the art. In some embodiments, the pharmaceutical compositions are in solid dosage forms, such as capsules, tablets, granules, powders, sachets, etc. In some embodiments, the pharmaceutical compositions are in the form of a sterile injectable solution in one or more aqueous or non-aqueous non-toxic parenterally-acceptable buffer systems, diluents, solubilizing agents, co-solvents, or carriers. A sterile injectable preparation may also be a sterile injectable aqueous or oily suspension or suspension in a non-aqueous diluent, carrier or co-solvent, which may be formulated according to known procedures using one or more of the appropriate dispersing or wetting agents and suspending agents. The pharmaceutical compositions could be a solution for iv bolus/infusion injection or a lyophilized system (either alone or with excipients) for reconstitution with a buffer system with or without other excipients. The lyophilized freeze-dried material may be prepared from non-aqueous solvents or aqueous solvents. The dosage form could also be a concentrate for further dilution for subsequent infusion.

The term "subject" includes warm-blooded mammals, for example, primates, dogs, cats, rabbits, rats, and mice. In some embodiments, the subject is a primate, for example, a human. In some embodiments, the subject is suffering from cancer, such as ovarian cancer, breast cancer, pancreatic cancer, and prostate cancer. In some embodiments the subject is suffering from cancer, such as hematological cancer, gastrointestinal such as gastric and colorectal cancer, or lung cancer. In some embodiments, the subject is suffering from ovarian cancer or breast cancer. In some embodiments, the subject is suffering from relapsed or refractory ovarian cancer. In some embodiments, the subject is suffering from relapsed or refractory breast cancer. In some embodiments, the subject is suffering from cancer and is treatment naïve (e.g., has never received treatment for cancer). In some embodiments, the subject is suffering from cancer and is platinum-resistant. Platinum-resistant disease is defined by progression within 6 months following the last administered platinum-based regimen. Platinumrefractory disease is defined by lack of at least a partial response while on platinum-containing regimens. A platinum-based regimen includes drugs that contain the metal platinum, such as cisplatin and carboplatin.

The language "therapeutically effective amount" includes that amount of AZD5305 and that amount of platinum chemotherapeutic agent which together will elicit a biological or medical response in a subject, for example, the reduction or inhibition of enzyme or protein activity related to PARP, or cancer; amelioration of symptoms of cancer; or the slowing or delaying of progression of cancer. In some embodiments, the language "therapeutically effective amount" includes the amount of AZD5305 and a platinum chemotherapeutic agent together that is effective to at least partially alleviate, inhibit, and/or ameliorate cancer or inhibit PARP, and/or reduce or inhibit the growth of a tumor or proliferation of cancerous cells in a subject. In some embodiments, the language "therapeutically effective amount" includes the amount of AZD5305 and a platinum chemotherapeutic agent together that is effective to at least partially reduce or inhibit the growth of a tumor or proliferation of cancerous cells in a subject.

In some embodiments, disclosed is a kit comprising: a first pharmaceutical composition comprising AZD5305 or a pharmaceutically acceptable salt thereof and a pharmaceutically acceptable carrier; and a second pharmaceutical composition comprising carboplatin or a pharmaceutically acceptable salt; and instructions for using the first and second pharmaceutical compositions in combination. In some embodiments, the first pharmaceutical composition comprises a first amount of AZD5305 or a pharmaceutically acceptable salt thereof, and the second pharmaceutical composition comprises a second amount of a platinum chemotherapeutic agent or a pharmaceutically acceptable salt thereof; wherein the first amount and the second amount together comprise a therapeutically effective amount.

### Examples

The combination therapy disclosed herein will now be further explained by reference to the following non-limiting examples.

### Example 1

### Preparation of AZD5305

### General Experimental Conditions

¹H NMR spectra were obtained using a Bruker 300 MHz, 400 MHz or 500 MHz spectrometer at 27 °C unless otherwise noted; chemical shifts are expressed in parts per million (ppm, δ units) and are referenced to the residual mono-¹H isotopologue of the solvent (CHCl₃: 7.24 ppm; CHDCl₂: 5.32 ppm; CD₃S(=O)CD₂H: 2.49 ppm). Coupling constants are given in units of hertz (Hz). Splitting patterns describe apparent multiplicities and are designated as s (singlet), d (doublet), t (triplet), q (quartet), m (multiplet) and br s (broad singlet). LC-MS was carried out using a Waters UPLC fitted with a Waters SQD mass spectrometer or Shimadzu LC-20AD LC-20XR LC-30AD with a Shimadzu 2020 mass spectrometer. Reported molecular ions correspond to [M+H]+ unless otherwise noted; for molecules with multiple isotopic patterns (Br, Cl, etc.) the reported value is the one obtained for the lowest isotope mass unless otherwise specified.

Flash chromatography was performed using straight phase flash chromatography on a SP1^{™} Purification system from Biotage^{™}, CombiFlash^{®}Rf from ISCO or on Gilson system from Thermo Fisher using normal phase silica FLASH+^{™} (40M, 25M or 12 M) or SNAP^{™} KP-Sil Cartridges (340, 100, 50 or 10), Flash Column silica-CS columns from Agela, with C18-flash columns or standard flash chromatography. In general, all solvents used were commercially available and of analytical grade. Anhydrous solvents were routinely used for reactions. Phase Separators used in the examples are ISOLUTE^{®} Phase Separator columns. The intermediates and examples named below were named using ACD/Name 12.01 from Advanced Chemistry Development, Inc. (ACD/Labs). The starting materials were obtained from commercial sources or made via literature routes.

XRPD analysis was performed using a Bruker D8 diffractometer, which is commercially available from Bruker AXS Inc^{™} (Madison, Wisconsin). The XRPD spectra were obtained by mounting a sample (approximately 10 mg) of the material for analysis on a single silicon crystal wafer mount (e.g., a Bruker silicon zero background X-ray diffraction sample holder) and spreading out the sample into a thin layer with the aid of a microscope slide. The sample was spun at 30 revolutions per minute (to improve counting statistics) and irradiated with X-rays generated by a copper long-fine focus tube operated at 40 kV and 40 mA with a wavelength of 1.5406 angstroms (i.e., about 1.54 angstroms). The sample was exposed for 1 second per 0.02 degree 2-theta increment (continuous scan mode) over the range 5 degrees to 40 degrees 2-theta in theta-theta mode. The running time was ~15 min for D8.

XRPD 2θ values may vary with a reasonable range, e.g., in the range ± 0.2° and that XRPD intensities may vary when measured for essentially the same crystalline form for a variety of reasons including, for example, preferred orientation. Principles of XRPD are described in publications, such as, for example, Giacovazzo, C. et al. (1995), Fundamentals of Crystallography, Oxford University Press; Jenkins, R. and Snyder, R. L. (1996), Introduction to X-Ray Powder Diffractometry, John Wiley & Sons, New York; and Klug, H. P. & Alexander, L. E. (1974), X-ray Diffraction Procedures, John Wiley and Sons, New York.

The following abbreviations are used: aq = aqueous; CH2Cl2 = dichloromethane; DCM = dichloromethane; DDQ = 2,3-Dichloro-5,6-dicyano-1,4-benzoquinone; DIPEA = *N,N-*diisopropylethylamine; DMSO = dimethylsulfoxide; DMSO-d₆ = deuterated dimethylsulfoxide; ESI = electrospray ionization; MeCN or CH3CN = acetonitrile; NMR = nuclear magnetic resonance; Pd/C = Palladium on carbonTFA = trifluoroacetic acid; THF = tetrahydrofuran; TLC = thin layer chromatography; HCl = Hydrochloric acid; HBr= Hydrobromic acid; Cs2CO3 = Cesium carbonate; MgSO4 = Magnesium sulfate; NaHCO3 = Sodium bicarbonate; SOCl2 = Thionyl chloride; NH₄Cl = ammonium chloride; Na₂SO₄ = sodium sulfate; H2 = hydrogen gas.

### Chemical Synthesis

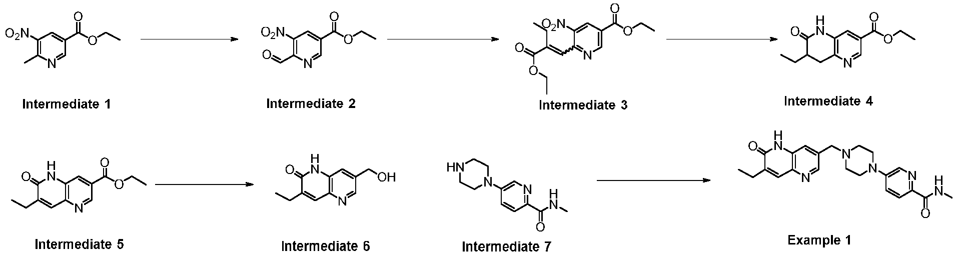

### Intermediate 2: ethyl 6-formyl-5-nitro-pyridine-3-carboxylate

A mixture of ethyl 6-methyl-5-nitro-pyridine-3-carboxylate (**intermediate 1,** 10 g, 47.58 mmol) and selenium dioxide (7.92 g, 71.36 mmol) in 1,4-dioxane (50 mL) was stirred at 110 °C for 20 h. The reaction mixture was cooled to room temperature, filtered through a pad of celite and the celite was washed with ethyl acetate. The combined filtrate was concentrated, and the resulting residue was purified by flash silica chromatography, elution gradient 0 to 70% ethyl acetate in hexanes. Product fractions were concentrated under reduced pressure to afford ethyl 6-formyl-5-nitro-pyridine-3-carboxylate (**Intermediate 2,** 9.70 g, 91 %) as a brown oil. 1H NMR (500 MHz, CHLOROFORM-d) 1.48 (3H, t), 4.54 (2H, q), 8.81 (1H, d), 9.51 (1H, d), 10.32 (1H, s); m/z (ES⁺) [M]⁺ = 224.

### Intermediate 3: ethyl 6-[(E)-2-ethoxycarbonylbut-1-enyl]-5-nitro-pyridine-3-carboxylate (mixture of E/Z isomers)

To a stirred solution of sodium hydride (9.63 g, 240.89 mmol) (60% in mineral oil) in anhydrous THF (100 mL) was added ethyl 2-(diethoxyphosphoryl)butanoate (60.8 g, 240.89 mmol) dropwise with an addition funnel at 0°C to give a grey colored mixture. The resulting mixture was stirred at 0°C for 10 min and warmed to room temperature over 10 minutes and stirred at 40°C for 5 minutes. The reaction mixture was cooled to -78°C and to this cooled reaction mixture was then slowly added solution of ethyl 6-formyl-5-nitro-pyridine-3-carboxylate (**Intermediate 2,** 22.5 g, 100.37 mmol) in 100 mL THF. The mixture was quenched with sat. NH₄Cl solution, extracted with ethyl acetate. The combined the organic layers were dried over sodium Na₂SO₄, filtered and concentrated to give crude product. the resulting residue was purified by flash silica chromatography, elution gradient 0 to 50% ethyl acetate in hexanes. Product fractions were concentrated under reduced pressure to afford ethyl 6-[(E)-2-ethoxycarbonylbut-1-enyl]-5-nitropyridine-3-carboxylate (**Intermediate 3**, 24.30 g, 75 %) as a yellow oil (1:1 and mixture of E/Z isomer). 1H NMR (500 MHz, CHLOROFORM-d) 1.13 (3H, t), 1.18 (3H, t), 1.23 (3H, t), 1.37 (3H, t), 1.45 (6H, q), 2.57 (2H, qd), 2.66 (2H, q), 4.11 - 4.24 (2H, m), 4.32 (2H, q), 4.45 - 4.56 (4H, m), 7.08 (1H, s), 7.85 (1H, s), 8.86 (2H, dd), 9.26 (1H, d), 9.43 (1H, d); m/z (ES⁺) [M]⁺ = 322

### Intermediate 4: ethyl 7-ethyl-6-oxo-7,8-dihydro-5H-1,5-naphthyridine-3-carboxylate

A mixture of ethyl 6-[(E)-2-ethoxycarbonylbut-1-enyl]-5-nitro-pyridine-3-carboxylate (1:1 mixture of E/Z isomers) (**Intermediate** 3, 3.75 g, 11.63 mmol), Pd/C (1.857g, 1.75 mmol) (10%) in ethanol (30 mL) was degassed, filled up with H2 (balloon), and the reaction was stirred at room temperature for overnight under H2 atmosphere. The mixture was filtered through a celite bed and the celite bed washed with ethanol. After concentration, 4M HCl in dioxanes (15 mL) was added to the resulting residue and the mixture was stirred at room temperature for 30 min. The mixture was diluted with ether and the solid was filtered off, washed with diethyl ether and dried under vacuum to afford ethyl 7-ethyl-6-oxo-7,8-dihydro-5H-1,5-naphthyridine-3-carboxylate (**Intermediate 4,** 2.260 g, 78 %) as a white solid. 1H NMR (500 MHz, DMSO-d6) 0.94 (3H, t), 1.33 (3H, t), 1.41 - 1.51 (1H, m), 1.69 - 1.81 (1H, m), 2.41 - 2.48 (1H, m), 2.94 (1H, dd), 3.20 (1H, dd), 4.35 (2H, t), 7.67 (1H, d), 8.61 (1H, d), 10.32 (1H, s); m/z (ES⁺) [M+H]⁺ = 249.

### Intermediate 5: ethyl 7-ethyl-6-oxo-5H-1,5-naphthyridine-3-carboxylate

Ethyl 7-ethyl-6-oxo-7,8-dihydro-5H-1,5-naphthyridine-3-carboxylate (**Intermediate 4,** 2.26 g, 9.10 mmol) was dissolve into 1,4-dioxane (40 mL), DDQ (2.273 g, 10.01 mmol) was added and the mixture was stirred at reflux for 3 h. Solvent was removed under reduced pressure, sat. NaHCO₃ solution was added and the residue stirred at room temperature for 1 hr. The solid was filtered off, washed with water followed by 10 mL of diethyl ether. The resulting solid was dried under vacuum afford ethyl 7-ethyl-6-oxo-5H-1,5-naphthyridine-3-carboxylate (**Intermediate 5,** 1.738 g, 78 %) as a light brown solid.

1H NMR (500 MHz, DMSO-d6) 1.14 - 1.28 (3H, m), 1.35 (3H, t), 2.58 (2H, q), 4.38 (2H, q), 7.83 (1H, s), 8.17 (1H, s), 8.90 (1H, s), 12.05 (1H, s); m/z (ES⁺) [M+H]⁺ = 247.

### Intermediate 6: 3-ethyl-7-(hydroxymethyl)-1H-1,5-naphthyridin-2-one

Lithium aluminum hydride, 2 M in THF (29.2 mL, 58.47 mmol) was added dropwise to ethyl 7-ethyl-6-oxo-5H-1,5-naphthyridine-3-carboxylate (**Intermediate 5,** 7.2 g, 29.24 mmol) in tetrahydrofuran (150 mL) at 0°C over a period of 45 minutes under nitrogen. The resulting mixture was stirred at 0 °C for 1.5 hours. The reaction mixture was quenched by dropwise addition of 1 M aq HCl (29 mL). The reaction mixture was concentrated and the solid was diluted with water (~ 150 mL) and 29 mL of 1M HCl solution gave a yellow suspension. The solid was collected by filtration, washed with water, diethyl ether and dried to yield the crude product as a yellow solid (contaminated by some inorganic salt). This solid was suspended in a mixture of methanol and DCM (2:1) (400 mL) and heated to reflux. The solid was filtered off. This solid was resuspended in methanol/DCM mixture and repeated this procedure 5 times to get most of the product out from this mixture. The combined filtrate was then concentrated until about 100 mL and the solid was collected by filtration, washed with ether, dried under vacuum to yield 3-ethyl-7-(hydroxymethyl)-1H-1,5-naphthyridin-2-one (**Intermediate 6,** 4.35 g, 72.8 %) as yellow solid. 1H NMR (500 MHz, DMSO-d6) 1.18 (3H, t), 2.52 - 2.56 (2H, m), 4.61 (2H, d), 5.44 (1H, t), 7.61 (1H, s), 7.74 (1H, s), 8.37 (1H, s), 11.87 (1H, br s); m/z (ES+) [M+H]+ = 205.3

### Example 1: 5-[4-[(7-ethyl-6-oxo-5H-1,5-naphthyridin-3-yl)methyl]piperazin-1-yl]-N-methylpyridine-2-carboxamide

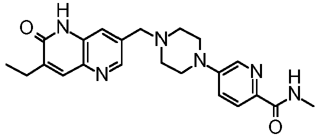

Thionyl chloride (6.41 mL, 88.14 mmol) was added dropwise to a suspension of 3-ethyl-7-(hydroxymethyl)-1,5-naphthyridin-2(1H)-one (**Intermediate 6,** 3 g, 14.69 mmol) and N,N-dimethylformamide (0.114 mL, 1.47 mmol) in CH2CI2 (60 mL) at 0°C and the resulting solution was stirred at room temperature for 6 hours. The mixture was concentrated to dryness to give crude 7-(chloromethyl)-3-ethyl-1H-1,5-naphthyridin-2-one.

DIPEA (12.83 mL, 73.45 mmol) was added to a stirred solution of 7-(chloromethyl)-3-ethyl-1H-1,5-naphthyridin-2-one (crude from above), potassium iodide (0.488 g, 2.94 mmol) and N-methyl-5-piperazin-1-yl-pyridine-2-carboxamide, 2HCl (**Intermediate 7,** 4.31 g, 14.69 mmol) in acetonitrile (50.00 mL) at 20°C. The resulting solution was stirred at 80 °C for 2 hours. Solvent was removed under vacuum. Crude material was diluted with water, basified with aq. NaHCO3 solution and extracted with ethyl acetate. Organic layer was dried over sodium sulphate and concentrated to give crude product. The resulting residue was purified by flash silica chromatography, elution gradient 0 to 15% MeOH in DCM. Product fractions were concentrated under reduced pressure to afford 5-[4-[(7-ethyl-6-oxo-5H-1,5-naphthyridin-3-yl)methyl]piperazin-1-yl]-N-methyl-pyridine-2-carboxamide (**Example 1,** 3.93 g, 65.8 %) as an off white partially crystalline solid. 1H NMR (500MHz, DMSO-d6) 1.19 (3H, t), 2.53 - 2.59 (6H, m), 2.79 (3H, d), 3.33 - 3.39 (4H, m), 3.66 (2H, s), 7.39 (1H, dd), 7.64 (1H, s), 7.76 (1H, s), 7.83 (1H, d), 8.27 (1H, d), 8.36 - 8.40 (1H, m), 8.41 (1H, d), 11.85 (1H, s); m/z (ES⁺) [M]⁺ = 406.

### Example 1 - Form A

In Example 1, 5-[4-[(7-ethyl-6-oxo-5H-1,5-naphthyridin-3-yl)methyl]piperazin-1-yl]-N-methylpyridine-2-carboxamide was obtained as a partially crystalline solid by evaporating a methanol/dichloromethane solution under reduced pressure. The crystalline material soobtained was characterised as crystalline Form A.

In the case of poor crystallinity, crystalline Form A was obtainable by suspending 20 mg of the crude sample in 0.20 ml of water, methanol, ethanol, acetone, acetonitrile, tetrahydrofuran, ethyl acetate or other solvent for 1 day at the ambient temperature or 50°C.

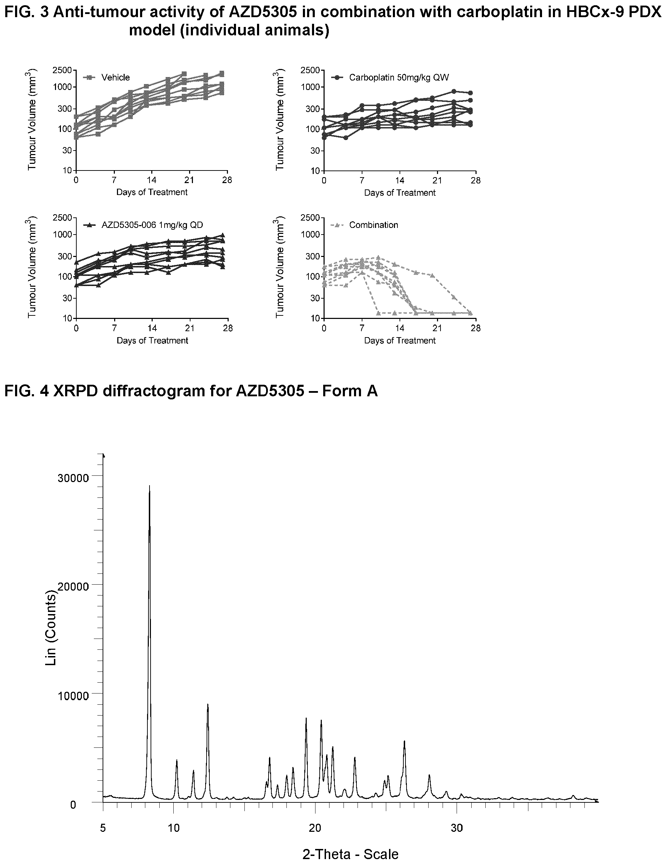

Form A was analysed by XRPD and the results are tabulated below (Table 1) and shown in **FIG 4****.**

**Table 1. XRPD Peaks for Form A**

| **Angle (2*θ*±0.2°)** | **Intensity (%)** |
|---|---|
| 8.3 | 100.0 |
| 12.4 | 30.9 |
| 19.4 | 26.5 |
| 20.4 | 25.8 |
| 26.3 | 19.2 |
| 21.2 | 17.4 |
| 20.8 | 14.8 |
| 22.8 | 14.1 |
| 16.8 | 14.0 |
| 10.2 | 13.2 |
| 18.4 | 10.8 |
| 11.4 | 9.9 |
| 28.1 | 8.4 |
| 18.0 | 8.4 |
| 25.2 | 8.2 |
| 24.9 | 6.7 |
| 16.5 | 6.4 |
| 17.3 | 5.3 |
| 22.1 | 4.0 |
| 29.3 | 3.3 |
| 24.3 | 2.7 |
| 30.3 | 2.5 |
| 38.2 | 2.0 |
| 33.9 | 1.4 |
| 14.2 | 1.4 |
| 13.7 | 1.4 |
| 33.0 | 1.3 |
| 36.5 | 1.2 |
| 39.2 | 1.2 |

Form A is characterized in providing at least one of the following 2*θ* values measured using CuKα radiation: 8.3, 12.4, and 19.4°.

### Example 2

This in vivo study was carried out to investigate the anti-tumour effects of AZD5305 in combination with carboplatin (a platinum chemotherapeutic agent) in a BRCA wild type tumour model, HBCx-9, in vivo. AZD5305 monotherapy and carboplatin monotherapy arms were included in the study design as controls.

### Materials and methods

### Abbreviations

| **Abbreviation or special term** | **Explanation** |
|---|---|
| 1N | 1 Molar |
| BRCA | Breast cancer susceptibility protein |
| BRCAm | BRCA mutated |
| CR | Complete response |
| HCl | Hydrochloric acid |
| HRD | Homologous recombination deoxyribonucleic acid pathway deficiency |
| NaCl | Sodium chloride |
| NaOH | Sodium hydroxide |
| PDX | Patient-derived tumour explant |
| PO | Oral(ly) |
| QD | Quaque die (once a day) |
| QD | Once a week |
| RB1 | Retinoblastoma transcriptional corepressor 1 |
| Reg | Regression |
| SC | Subcutaneous |
| SEM | Standard error of the mean |
| SLFN11 | Schlafen family member 11 |
| TGI | Tumour growth inhibition |
| TNBC | Triple negative breast cancer |
| TV | Tumour volume |
| WT | Wild-type |

### Live phase

HBCx-9 is a patient-derived tumour explant (PDX) model established in XenTech without prior in vitro culture. Tumour fragments were transplanted subcutaneously (SC) onto donor mice. When tumour volume reached 700 to 1764 mm³, donor mice were sacrificed, and tumours were aseptically excised and dissected. After removing necrotic areas, tumours were cut into fragments measuring approximately 20 mm³ and transferred in culture medium before grafting SC into the recipient (experimental) Nude female mice. Tumours were measured (length x width) two times weekly by bilateral Vernier calliper measurements and tumour volume was calculated using elliptical formula (π/6×width×width×length). Animal bodyweight and tumour condition were monitored throughout the study. Mice were randomised into treatment groups when mean tumour volume reached approximately 100 mm³. Animals were treated from the day after the randomisation. Control animals were treated orally (PO) once daily (QD) with vehicle (deionised water acidified with HCl to pH 3.5-4). AZD5305 was dosed PO QD at 1 mg/kg. Carboplatin was dosed intraperitoneally (IP) once weekly (QW) at 50 mg/kg. In the combination group, mice were dosed with carboplatin first, followed by AZD5305 administration within 10 minutes.

Tumour growth inhibition from start of treatment was assessed by comparison of the mean change in tumour volume of the control and treated groups and represented as percent tumour growth inhibition (TGI, when TV ≥starting TV) or regression (reg, when TV <starting TV). Mean percent body weight change from start of treatment was also calculated for all the groups. Statistical significance was evaluated using a one-tailed t test. Statistical significance is indicated as follows: * p ≤0.05, ** p ≤0.01, *** p ≤0.001.

Tolerance studies using AZD5305 at 10 mg/kg PO QD in combination with carboplatin at 50 mg/kg IP QWwere run in SCID female mice prior to commencement of the efficacy study and showed no adverse effects to animal condition or significant changes to bodyweight.

### Formulation of test materials

AZD5305 was formulated for oral dosing in water/HCl pH3.5-4 vehicle to be administered at 0.1 ml/10g dose volume. Solid was diluted by adding appropriate volume of 1N HCl to about 1:1.25 drug/HCl molar ratio and 80% of final volume of sterile deionized water with stirring/vortex/sonication until a solution was obtained. The required volume of 1N HCl was calculated according to the following equation: Volume of 1N HCl added (ml) = 1.25 × [concentration (mg/ml) ÷ 406.48 (g/mole) ÷ 1M] × final volume (ml)

The pH of the solution was adjusted to 3.5 to 4 with 1N HCl or 1N NaOH. The final volume was made up using sterile water for injections or deionized water and the final pH was measured and recorded. Dosing preparations were formulated once per week and protected from light. Carboplatin (Teva^{®}) was formulated fresh on each day of dosing by diluting the stock solution in 0.9% NaCl to 5 mg/ml for 50 mg/kg dose (0.1 ml/10g dose volume).

### RESULTS

HBCx-9 is a TNBC PDX model available in XenTech, France. It has been reported as BRCA wt model, with BRCA1 methylation [1]. HBCx-9 was also shown to have a moderate sensitivity to olaparib monotherapy [2].

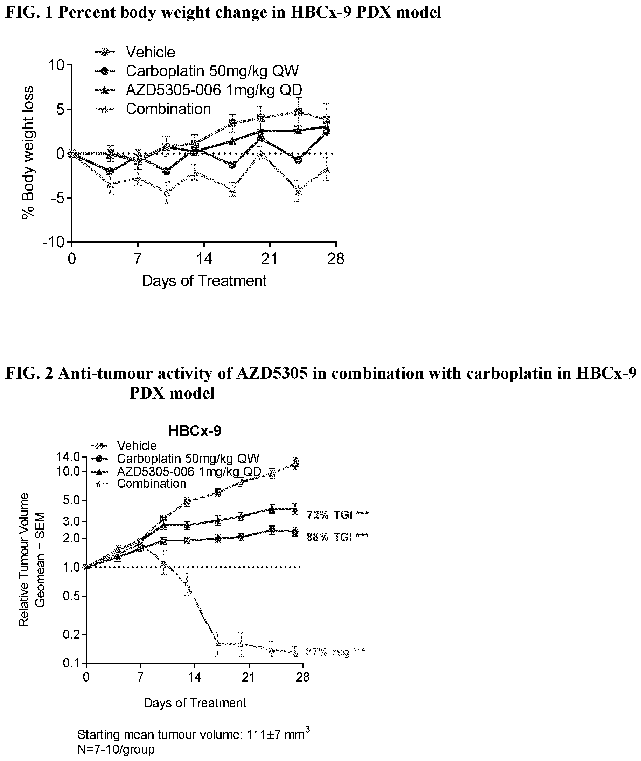

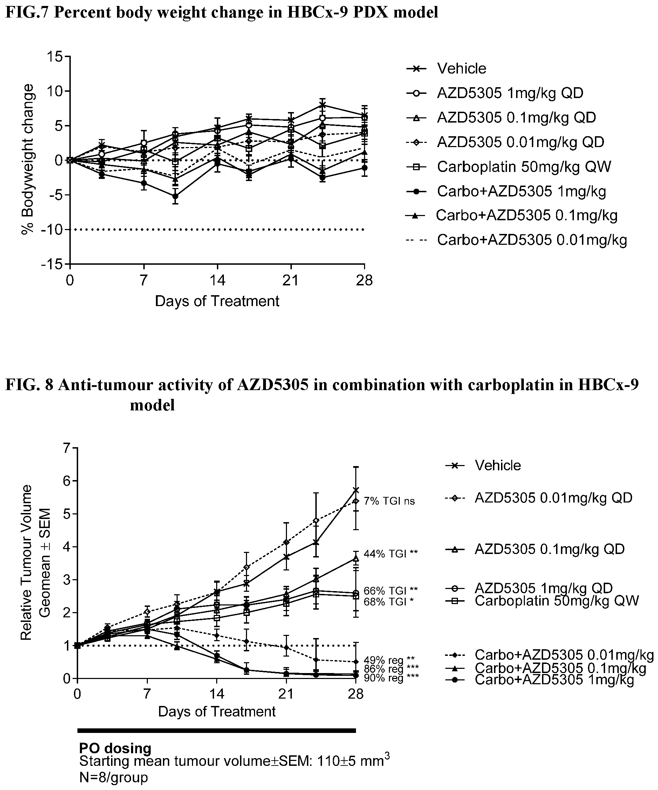

AZD5305 at 1, 0.1, and 0.01 mg/kg QD and carboplatin at 50 mg/kg QW were tested in the efficacy study as monotherapies and in combination. The treatment period lasted for 28 days. All treatments were well tolerated throughout the duration of the study and no significant body weight loss was observed (Figures 1 &7). AZD5305 monotherapy showed dose-dependent anti-tumour efficacy, delivering 72% TGI and 88% TGI was observed in carboplatin monotherapy group. AZD5305 and carboplatin dosed together demonstrated clear combination benefit by delivering 88% tumour regression (Figure 2 and Table 2). Moreover, all the animals in the combination group (8/8) reached a complete response (CR; defined as TV<14mm³) (Figure 3).

In conclusion, AZD5305 in combination with carboplatin demonstrated improved anti-tumour effect compared to each monotherapy in HBCx-9 PDX model in vivo. Combination of AZD5305 and carboplatin led to complete responses in 100% of animals and was well tolerated in mice.

**Table 2: in vivo anti-tumour effects of AZD5305, carboplatin and combination of both agents in HBCx-9 patient-derived explant model.**

| **Tumour Model** | **Treatment** | **Dose and Schedule** | **Duration of Dosing (days)** | **Tumour Growth Inhibition/Regression (end of treatment) (p-value t-test vs vehicle control)** |
|---|---|---|---|---|
| HBCx-9 | AZD5305 | 1 mg/kg QD | 28 | 72% TGI *** (p ≤0.001) |
| | Carboplatin | 50 mg/kg QW | | 88% TGI *** (p ≤0.001) |
| | AZD5305 + carboplatin | as above | | 87% regression *** (p ≤0.001) |

| | | | | |
|---|---|---|---|---|
| [1] Coussy F, de Koning L, Lavigne M, et al. A large collection of integrated genomically characterized patient-derived xenografts highlighting the heterogeneity of triple-negative breast cancer. Int J Cancer. 2019;145(7):1902-1912. doi:10.1002/ijc.32266 [2] LC Riches (2020) Mol Cancer Ther 2020;19:13-25 | | | | |

### Example 3

This study was carried out to investigate the anti-tumour effects of AZD5305 in combination with carboplatin in a BRCA1 mutant tumour model, SUM149PT, in vivo. AZD5305 monotherapy and carboplatin monotherapy arms were included in the study design as controls.

### MATERIALS AND METHODS

SUM149PT cells (2 × 10⁶) were implanted with 50% Matrigel into mammary fat pad (MFP) of female SCID mice (weight greater than 18 g). Tumours were measured (length x width) two times weekly by bilateral Vernier calliper measurements and tumour volume was calculated using elliptical formula (π/6×width×width×length). Animal bodyweight and tumour condition were monitored throughout the studies. Mice were randomised into treatment groups when mean tumour volume reached approximately 0.3 cm³. Animals were treated from the day after the randomisation. Control animals were treated orally (PO) once daily (QD) with vehicle (deionised water acidified with HCl to pH 3.5-4) and intraperitoneally (IP) once weekly (QW) with PBS. AZD5305 was dosed PO QD at 0.1, 0.03 or 0.01 mg/kg. Carboplatin was dosed IP once weekly (QW) at 37.5 mg/kg. In the combination group, mice were dosed with carboplatin first, followed by AZD5305 administration within 10 minutes.

Tumour growth inhibition from start of treatment was assessed by comparison of the mean change in tumour volume of the control and treated groups and represented as percent tumour growth inhibition (TGI, when TV ≥starting TV) or regression (reg, when TV <starting TV). Mean percent body weight change from start of treatment was also calculated for all the groups. Statistical significance was evaluated using a one-tailed t test. Statistical significance is indicated as follows: * p ≤0.05, ** p --<0.01, *** p --<0.001.

Tolerance studies using AZD5305 at 10 mg/kg PO QD in combination with carboplatin at 50 mg/kg IP QWwere run in SCID female mice prior to commencement of the efficacy study and showed no adverse effects to animal condition or significant changes to bodyweight.

### Formulation of test materials

AZD5305 was formulated for oral dosing in water/HCl pH3.5-4 vehicle to be administered at 0.1 ml/10 g dose volume. Solid was diluted by adding appropriate volume of 1N HCl to about 1:1.25 drug/HCl molar ratio and 80% of final volume of sterile deionized water with stirring/vortex/sonication until a solution was obtained. The required volume of 1N HCl was calculated according to the following equation: Volume of 1N HCl added (ml) = 1.25 × [concentration (mg/ml) ÷ 406.48 (g/mole) ÷ 1M] × final volume (ml)

The pH of the solution was adjusted to 3.5-4 with 1N HCl or 1N NaOH. The final volume was made up using sterile water for injections or deionized water and the final pH was measured and recorded. Dosing preparations were formulated once per week and protected from light. Carboplatin (Sigma Aldrich) was formulated fresh on each day of dosing by diluting the stock solution in 0.9% NaCl to 3.75 mg/ml for 37.5 mg/kg dose (0.1 ml/10 g dose volume).

### RESULTS

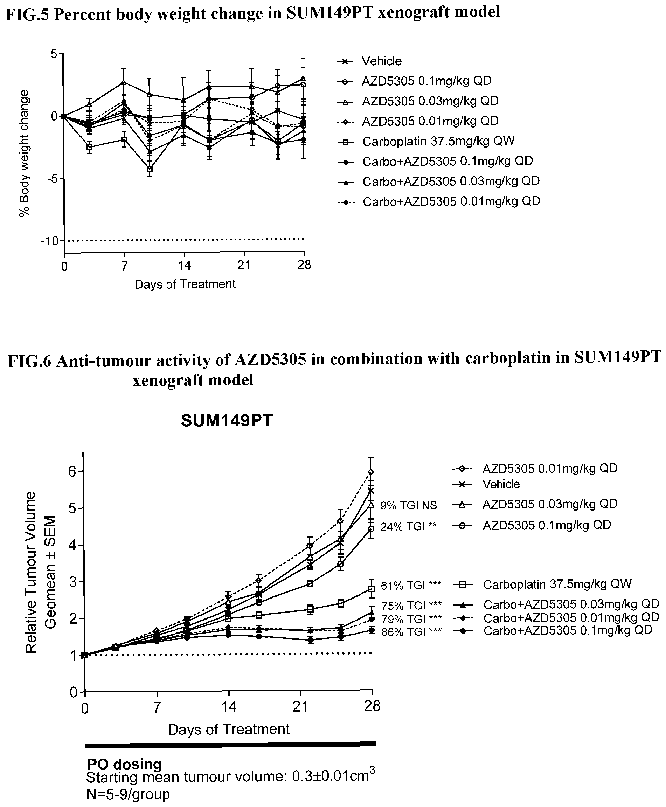

AZD5305 at 0.1, 0.03 and 0.01 mg/kg QD and carboplatin at 37.5 mg/kg QW were tested in the efficacy study in SUM149PT TNBC BRCA1m xenograft model as monotherapies and in combination. All treatments were well tolerated throughout the duration of the study (28 days) and no significant body weight loss was observed (Figure 5). AZD5305 monotherapy showed dose-dependent anti-tumour efficacy, delivering 24% TGI and 9% TGI at 0.1 mg/kg QD and 0.03 mg/kg QD, respectively. AZD5305 dosed at 0.01 mg/kg QD did not inhibit tumour growth. Carboplatin monotherapy resulted in 61% TGI. Combination of 0.1 mg/kg AZD5305 and carboplatin demonstrated a combination benefit and delivered 86% TGI. This anti-tumour effect was maintained even when dose level of AZD5305 in the combination with carboplatin was reduced to 0.03 mg/kg or 0.01 mg/kg (75% TGI and 79% TGI, respectively) (Figure 6 and Table 3).

In conclusion, AZD5305 in combination with carboplatin demonstrated improved anti-tumour effect compared to monotherapy groups in SUM149PT xenograft model in vivo. Decreasing dose level of AZD5305 by 10-fold (from 0.1 mg/kg to 0.01 mg/kg) in combination with carboplatin did not affect the tumour growth inhibition effect.

**Table 3 Summary of in vivo anti-tumour effects of AZD5305, carboplatin and combination of both agents in SUM149PT xenograft model.**

| **Tumour Model** | **Treatment** | **Dose and Schedule** | **Duration of Dosing (days)** | **Tumour Growth Inhibition/Regression (end of treatment) (p-value t-test vs vehicle control)** |
|---|---|---|---|---|
| SUM149PT | Carboplatin | 37.5 mg/kg QW | 28 | 61% TGI *** (p ≤0.001) |
| | AZD5305 | 0.1 mg/kg QD | | 24% TGI ** (p ≤0.01) |
| | AZD5305 | 0.03 mg/kg QD | | 9% TGI (not significant) |
| | AZD5305 | 0.01 mg/kg QD | | No TGI or regression |
| | Carboplatin + AZD5305 | 37.5 mg/kg QD + 0.1 mg/kg QD | | 86% TGI *** (p ≤0.001) |
| | Carboplatin + AZD5305 | 37.5 mg/kg QD + 0.03 mg/kg QD | | 75% TGI *** (p ≤0.001) |
| | Carboplatin + AZD5305 | 37.5 mg/kg QD + 0.01 mg/kg QD | | 79% TGI *** (p ≤0.001) |

### Example 4

This study was carried out to investigate the anti-tumour effects of a dose range of AZD5305 in combination with carboplatin in a BRCA1/2 wild type tumour model, HBCx-9, in vivo. AZD5305 monotherapy and carboplatin monotherapy arms were included in the study design as controls.

### MATERIALS AND METHODS

HBCx-9 is a patient-derived tumour explant (PDX) model established in XenTech without prior in vitro culture. Tumour fragments were transplanted subcutaneously (SC) onto donor mice. When tumour volume reached 1008 to 1764 mm3, donor mice were sacrificed, and tumours were aseptically excised and dissected. After removing necrotic areas, tumours were cut into fragments measuring approximately 20 mm3 and transferred in culture medium before grafting SC into the recipient (experimental) Nude female mice. Tumours were measured (length x width) two times weekly by bilateral Vernier calliper measurements and tumour volume was calculated using elliptical formula (π/6×width×width×length). Animal bodyweight and tumour condition were monitored throughout the study. Mice were randomised into treatment groups when mean tumour volume reached approximately 110 mm3. Animals were treated from the day after the randomisation. Control animals were treated orally (PO) once daily (QD) with vehicle (deionised water acidified with HCl to pH 3.5-4). AZD5305 was dosed PO QD at 1, 0.1, or 0.01 mg/kg. Carboplatin was dosed IP once weekly (QW) at 50 mg/kg. In the combination group, mice were dosed with carboplatin first, followed by AZD5305 administration within 10 minutes.

Tumour growth inhibition from start of treatment was assessed by comparison of the mean change in tumour volume of the control and treated groups and represented as percent tumour growth inhibition (TGI, when TV ≥starting TV) or regression (reg, when TV <starting TV). Mean percent body weight change from start of treatment was also calculated for all the groups. Statistical significance was evaluated using a one-tailed t test. Statistical significance is indicated as follows: * p ≤0.05, ** p --<0.01, *** p --<0.001.

Tolerance studies using AZD5305 at 10 mg/kg PO QD in combination with carboplatin at 50 mg/kg IP QW were run in SCID female mice prior to commencement of the efficacy study and showed no adverse effects to animal condition or significant changes to bodyweight.

Samples for plasma PK were obtained by collecting 400-600 µL of whole blood by intra-cardiac puncture under xylazine-ketamine anaesthesia. Blood was transferred into BD Microtainer Hep-Li with separator gel tubes and centrifuged at 5000 RPM for 5-10 minutes at 4°C. Separated plasma was collected and stored at -80°C. To determine compound levels in plasma samples, each plasma sample (25 µl) was prepared using an appropriate dilution factor and compared against an 11-point standard calibration curve (1-10000 nM) prepared in DMSO and spiked into blank plasma. Acetonitrile (100 µl) was added with the internal standard, followed by centrifugation at 3000 RPM for 10 minutes. Supernatant (50 µl) was then diluted in 300 µl water and analysed via UPLC-MS/MS.

### Formulation of test materials

AZD5305 was formulated for oral dosing in water/HCl pH3.5-4 vehicle to be administered at 0.1 ml/10 g dose volume. Solid was diluted by adding appropriate volume of 1N HCl to about 1:1.25 drug/HCl molar ratio and 80% of final volume of sterile deionized water with stirring/vortex/sonication until a solution was obtained. The required volume of 1N HCl was calculated according to the following equation: Volume of 1N HCIl added (ml) = 1.25 × [concentration (mg/ml) ÷ 406.48 (g/mole) ÷ 1M] × final volume (ml)

The pH of the solution was adjusted to 3.5-4 with 1N HCl or 1N NaOH. The final volume was made up using sterile water for injections or deionized water and the final pH was measured and recorded. Dosing preparations were formulated once per week and protected from light. Carboplatin (Sandoz) was formulated fresh on each day of dosing by diluting the stock solution in 0.9% NaCl to 5 mg/ml for 50 mg/kg dose (0.1 ml/10 g dose volume).

### RESULTS

HBCx-9 is a TNBC PDX model available in XenTech, France. It has been reported as BRCA1/2 wt model with BRCA1 methylation, as well as "BRCAness features" [1]. HBCx-9 was also shown to have a moderate sensitivity to olaparib monotherapy [2].

AZD5305 at 1, 0.1, and 0.01 mg/kg QD and carboplatin at 50 mg/kg QW were tested in the efficacy study as monotherapies and in combination. All treatments were well tolerated throughout the duration of the study (28 days) and no significant body weight loss was observed. AZD5305 monotherapy showed dose-dependent anti-tumour efficacy, delivering 66%, 44%, and 7% TGI at 1, 0.1, and 0.01 mg/kg QD, respectively. Carboplatin monotherapy resulted in 68% TGI. Treatment with 1 mg/kg AZD5305 and carboplatin demonstrated a combination benefit and delivered 90% regression. This anti-tumour effect was maintained even when dose level of AZD5305 in the combination with carboplatin was reduced to 0.1 mg/kg (86% regression). The effect was slightly reduced when carboplatin was combined with 0.01 mg/kg of AZD5305 (49% TGI). However, even this group deliver a combination benefit comparing to either monotherapy arm.

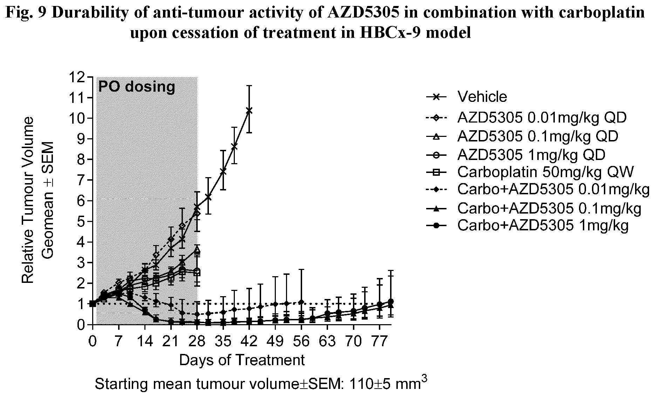

In this experiment the treatment was continued for 28 days and then the duration of response was monitored off treatment. The graph in figure 9 depicts that in the most efficacious groups (carboplatin combined with AZD5305 at 1 or 0.1 mg/kg) tumours started to regrow around day 49 (3 weeks after cessation of treatment), and the rate of the regrowth was similar between the two groups. Additionally, in carboplatin + 0.01 mg/kg of AZD5305 group tumours started to regrow about 2 weeks after treatment withdrawal.

In conclusion, AZD5305 in combination with carboplatin demonstrated improved anti-tumour effect compared to monotherapy groups in HBCx-9 PDX model in vivo. Decreasing dose level of AZD5305 by 10-fold (from 1 mg/kg to 0.1 mg/kg) in combination with carboplatin did not affect the tumour regression effect.

**Table 4 Summary of in vivo anti-tumour effects of AZD5305, carboplatin and combination of both agents in HBCx-9 model.**

| **Tumour Model** | **Treatment** | **Dose and Schedule** | **Duration of Dosing (days)** | **Tumour Growth Inhibition/Regression (end of treatment) (p-value t-test vs vehicle control)** |
|---|---|---|---|---|
| HBCx-9 | Carboplatin | 50 mg/kg QW | 28 | 68% TGI * (p ≤0.05) |
| | AZD5305 | 1 mg/kg QD | | 66% TGI ** (p ≤0.01) |
| | AZD5305 | 0.1 mg/kg QD | | 44% TGI ** (p ≤0.01) |
| | AZD5305 | 0.01 mg/kg QD | | 7% TGI (not significant) |
| | Carboplatin + AZD5305 | 50 mg/kg QD + 1 mg/kg QD | | 90% regression *** (p ≤0.001) |
| | Carboplatin + AZD5305 | 50 mg/kg QD + 0.1 mg/kg QD | | 86% regression *** (p ≤0.001) |
| | Carboplatin + AZD5305 | 50 mg/kg QD + 0.01 mg/kg QD | | 49% TGI ** (p ≤0.01) |

This written description uses examples to disclose the invention and to enable any person skilled in the art to practice the invention, including making and using any of the disclosed salts, substances, or compositions, and performing any of the disclosed methods or processes. The patentable scope of the invention is defined by the claims. While preferred embodiments of the invention are shown and described in this specification, such embodiments are provided by way of example only and are not intended to otherwise limit the scope of the invention.

## Claims

1. AZD5305, or a pharmaceutically acceptable salt thereof, for use in the treatment of cancer in a subject, wherein said treatment comprises the separate, sequential or simultaneous administration of i) said AZD5305, or a pharmaceutically acceptable salt thereof, and ii) a platinum chemotherapeutic agent, or a pharmaceutically acceptable salt thereof, to said subject.

2. The compound for use
according to claim 1, wherein the cancer is selected from ovarian cancer breast cancer, pancreatic cancer, prostate cancer, hematological cancer, gastrointestinal cancer such as gastric cancer and colorectal cancer, and lung cancer.

3. The compound for use
according to claim 1 or claim 2, wherein the platinum chemotherapeutic agent is selected from any one of cisplatin, oxaliplatin, and carboplatin.

4. The compound for use
according to any one of claims 1 to 3, wherein the cancer is homologous recombination deficient (HRD) cancer.

5. The compound for use
according to claim 4, wherein the cancer comprises cells having a mutation in an HRR gene selected from *BRCA1, BRCA2, ATM, BRIP1, BARD1, CDK12, CHEK1, CHEK2, FANCL, PALB2, PPP2R2A, RAD51B, RAD51C, RAD51D,* and *RAD54L.*

6. The compound for use
according to claim 5, wherein the mutated HRR gene is selected from *BRCA1, BRCA2,* and *ATM.*

7. A pharmaceutical product comprising i) AZD5305 or a pharmaceutically acceptable salt thereof, and ii) a platinum chemotherapeutic agent or a pharmaceutically acceptable salt thereof.

8. The pharmaceutical product according to claim 7, wherein AZD5305 or a pharmaceutically acceptable salt thereof and the platinum chemotherapeutic agent or a pharmaceutically acceptable salt thereof are present in a single dosage form.

9. The pharmaceutical product according to claim 7, wherein AZD5305 or a pharmaceutically acceptable salt thereof and the platinum chemotherapeutic agent or a pharmaceutically acceptable salt thereof are present in separate dosage forms.

10. The compound for use
or pharmaceutical composition according to any preceding claim wherein the platinum chemotherapeutic agent comprises carboplatin.

11. The compound for use
or pharmaceutical composition according to any preceding claim wherein the platinum chemotherapeutic agent is carboplatin.

12. The compound for use
or pharmaceutical composition according to any preceding claim wherein the AZD5305 is the free base.

13. A kit comprising:
a first pharmaceutical composition comprising AZD5305 or a pharmaceutically acceptable salt thereof and a pharmaceutically acceptable carrier; and
a second pharmaceutical composition comprising a platinum chemotherapeutic agent or a pharmaceutically acceptable salt thereof and a pharmaceutically acceptable carrier, and instructions for use.

## Patentansprüche

1. AZD5305 oder pharmazeutisch annehmbares Salz davon zur Verwendung bei der Behandlung von Krebs in einem Subjekt, wobei die Behandlung die separate, aufeinanderfolgende oder gleichzeitige Verabreichung i) des AZD5305 oder eines pharmazeutisch annehmbaren Salzes davon und ii) eines Platin-Chemotherapeutikums oder eines pharmazeutisch annehmbaren Salzes davon an das Subjekt umfasst.

2. Verbindung zur Verwendung nach Anspruch 1, wobei der Krebs aus Eierstockkrebs, Brustkrebs, Bauchspeicheldrüsenkrebs, Prostatakrebs, hämatologischem Krebs, Magen-Darm-Krebs wie Magenkrebs und Darmkrebs sowie Lungenkrebs ausgewählt ist.

3. Verbindung zur Verwendung nach Anspruch 1 oder Anspruch 2, wobei das Platin-Chemotherapeutikum aus Cisplatin, Oxaliplatin und Carboplatin ausgewählt ist.

4. Verbindung zur Verwendung nach einem der Ansprüche 1 bis 3, wobei es sich bei dem Krebs um Krebs mit defekter homologer Rekombination (Homologous Recombination Deficient Cancer, HRD Cancer) handelt.

5. Verbindung zur Verwendung nach Anspruch 4, wobei der Krebs Zellen mit einer Mutation in einem HRR-Gen ausgewählt aus BRCA1, BRCA2, ATM, BRIP1, BARD1, CDK12, CHEK1, CHEK2, FANCL, PALB2, PPP2R2A, RAD51B, RAD51C, RAD51D und RAD54L umfasst.

6. Verbindung zur Verwendung nach Anspruch 5, wobei das mutierte HRR-Gen aus BRCA1, BRCA2 und ATM ausgewählt ist.

7. Pharmazeutisches Produkt, umfassend i) AZD5305 oder ein pharmazeutisch annehmbares Salzes davon und ii) ein Platin-Chemotherapeutikums oder ein pharmazeutisch annehmbares Salzes davon.

8. Pharmazeutisches Produkt nach Anspruch 7, wobei AZD5305 oder ein pharmazeutisch annehmbares Salzes davon und das Platin-Chemotherapeutikum oder ein pharmazeutisch annehmbares Salzes davon in einer einzigen Verabreichungsform vorliegen.

9. Pharmazeutisches Produkt nach Anspruch 7, wobei AZD5305 oder ein pharmazeutisch annehmbares Salzes davon und das Platin-Chemotherapeutikum oder ein pharmazeutisch annehmbares Salzes davon in separaten Verabreichungsform vorliegen.

10. Verbindung zur Verwendung oder pharmazeutische Zusammensetzung nach einem der vorhergehenden Ansprüche, wobei das Platin-Chemotherapeutikum Carboplatin umfasst.

11. Verbindung zur Verwendung oder pharmazeutische Zusammensetzung nach einem der vorhergehenden Ansprüche, wobei es sich bei dem Platin-Chemotherapeutikum um Carboplatin handelt.

12. Verbindung zur Verwendung oder pharmazeutische Zusammensetzung nach einem der vorhergehenden Ansprüche, wobei es sich bei dem AZD5305 um die freie Base handelt.

13. Kit, umfassend:
eine erste pharmazeutische Zusammensetzung, die AZD5305 oder ein pharmazeutisch annehmbares Salzes davon und
einen pharmazeutisch annehmbaren Träger umfasst, und
eine zweite pharmazeutische Zusammensetzung, die ein Platin-Chemotherapeutikum oder ein pharmazeutisch annehmbares Salzes davon und einen pharmazeutisch annehmbaren Träger umfasst, sowie Gebrauchsanweisungen.

## Revendications

1. AZD5305, ou sel pharmaceutiquement acceptable correspondant, pour une utilisation dans le traitement d'un cancer chez un sujet, ledit traitement comprenant l'administration séparée, séquentielle ou simultanée i) dudit AZD5305, ou d'un sel pharmaceutiquement acceptable correspondant, et ii) d'un agent chimiothérapeutique au platine, ou d'un sel pharmaceutiquement acceptable correspondant, audit sujet.

2. Composé pour une utilisation selon la revendication 1, le cancer étant choisi parmi un cancer de l'ovaire, un cancer du sein, un cancer pancréatique, un cancer de la prostate, un cancer hématologique, un cancer gastro-intestinal tel qu'un cancer gastrique et un cancer colorectal, et un cancer du poumon.

3. Composé pour une utilisation selon la revendication 1 ou la revendication 2, l'agent chimiothérapeutique au platine étant choisi parmi l'un quelconque parmi le cisplatine, l'oxaliplatine et le carboplatine.

4. Composé pour une utilisation selon l'une quelconque des revendications 1 à 3, le cancer étant un cancer déficient en recombinaison homologue (HRD).

5. Composé pour une utilisation selon la revendication 4, le cancer comprenant des cellules ayant une mutation dans un gène HRR choisi parmi *BRCA1, BRCA2, ATM, BRIP1, BARD1, CDK12, CHEK1, CHEK2, FANCL, PALB2, PPP2R2A, RAD51B, RAD51C, RAD51D,* et *RAD54L.*

6. Composé pour une utilisation selon la revendication 5, le gène HRR muté étant choisi parmi *BRCA1, BRCA2,* et *ATM.*

7. Produit pharmaceutique comprenant i) AZD5305, ou un sel pharmaceutiquement acceptable correspondant, et ii) un agent chimiothérapeutique au platine, ou un sel pharmaceutiquement acceptable correspondant.

8. Produit pharmaceutique selon la revendication 7, AZD5305, ou un sel pharmaceutiquement acceptable correspondant, et l'agent chimiothérapeutique au platine, ou un sel pharmaceutiquement acceptable correspondant étant présents dans une forme de dosage unique.

9. Produit pharmaceutique selon la revendication 7, AZD5305, ou un sel pharmaceutiquement acceptable correspondant, et l'agent chimiothérapeutique au platine, ou un sel pharmaceutiquement acceptable correspondant étant présents dans des formes de dosage distinctes.

10. Composé pour une utilisation ou composition pharmaceutique selon une quelconque revendication précédente, l'agent chimiothérapeutique au platine comprenant le carboplatine.

11. Composé pour une utilisation ou composition pharmaceutique selon une quelconque revendication précédente, l'agent chimiothérapeutique au platine étant le carboplatine.

12. Composé pour une utilisation ou composition pharmaceutique selon une quelconque revendication précédente, l'AZD5305 étant la base libre.

13. Kit comprenant :
une première composition pharmaceutique comprenant AZD5305 ou un sel pharmaceutiquement acceptable correspondant et un support pharmaceutiquement acceptable ; et
une deuxième composition pharmaceutique comprenant un agent chimiothérapeutique au platine ou un sel pharmaceutiquement acceptable correspondant et un support pharmaceutiquement acceptable, et des instructions pour une utilisation.
